# EUROPEAN PATENT APPLICATION

(11) **EP 2 166 016 A1**
(43) Date of publication of application: **24.03.2010**
(21) Application number: 08164615.0
(22) Date of filing: 18.09.2008
(51) Int. Cl.: C07H 19/10, A61K 31/7068, A61P 31/14

(54) **Phosphoramidate Derivatives of Nucleosides**

(71) Applicant: Centocor Ortho Biotech Products L.P., Bridgewater, NJ 08807 (US); Medivir AB, 141 44 Huddinge (SE)
(72) Inventor: Jonckers, Tim Hugo Maria, B-2650 Edegem (BE); Nilsson, Karl Magnus, S-141 44 Huddinge (SE); Raboisson, Pierre Jean-Marie Bernard, B-1331 Rosieres (BE)
(74) Representative: Wante, Dirk Paul Maria

(57) **Abstract**

Compounds of formula I : 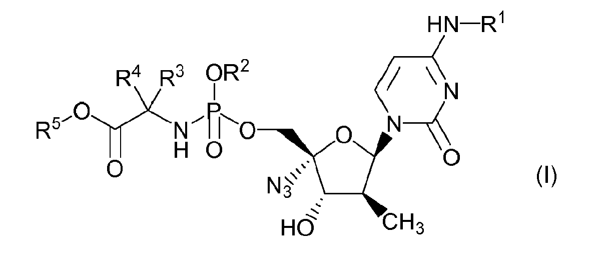 R¹ is hydrogen, C₁-C₄alkyl, OH, C₁-C₄alkoxy;
R² is phenyl, optionally substituted with 1, 2 or 3 substituents each independently selected from halo, C₁-C₆alkyl, and C₁-C₆alkoxy, or R² is naphtalenyl;
R³ is hydrogen, C₁-C₆alkyl, benzyl;
R⁴ is hydrogen, C₁-C₆alkyl, benzyl; or
R³ and R⁴ together with the carbon atom to which they are attached form C₃-C₇cycloalkyl;
R⁵ is C₁-C₆alkyl, benzyl, or phenyl, optionally substituted with 1, 2 or 3 substituents each independently selected from hydroxy, C₁-C₆alkoxy, amino, mono- and diC₁-C₆alkylamino;
or a pharmaceutically acceptable salt or solvate thereof;
useful in the prophylaxis or treatment of HCV infections.

## Description

### Field of the Invention

This invention relates to novel nucleoside compounds, which are inhibitors of the polymerase of hepatitis C virus (HCV) and their use in the treatment or prophylaxis of HCV.

### Background of the Invention

HCV is a single stranded, positive-sense RNA virus, with a genome of around 9,600 bases belonging to the *Flaviviridae* family of viruses in the hepacivirus genus. The NS5B region of the RNA polygene encodes a 65 kDa RNA dependent RNA polymerase (RdRp), which is essential to viral replication. Following the initial acute infection, a majority of infected individuals develop chronic hepatitis because HCV replicates preferentially in hepatocytes but is not directly cytopathic. In particular, the lack of a vigorous T-lymphocyte response and the high propensity of the virus to mutate appear to promote a high rate of chronic infection. Chronic hepatitis can progress to liver fibrosis, leading to cirrhosis, end-stage liver disease, and HCC (hepatocellular carcinoma), making it the leading cause of liver transplantations.

There are six major HCV genotypes and more than 50 subtypes, which are differently distributed geographically. HCV genotype 1 is the predominant genotype in Europe and the US. The extensive genetic heterogeneity of HCV has important diagnostic and clinical implications, perhaps explaining difficulties in vaccine development and the lack of response to current therapy.

Transmission of HCV can occur through contact with contaminated blood or blood products, for example following blood transfusion or intravenous drug use. The introduction of diagnostic tests used in blood screening has led to a downward trend in post-transfusion HCV incidence. However, given the slow progression to the end-stage liver disease, the existing infections will continue to present a serious medical and economic burden for decades.

Current HCV therapies are based on (pegylated) interferon-alpha (IFN-α) in combination with ribavirin. This combination therapy yields a sustained virologic response in more than 40% of patients infected by genotype 1 viruses and about 80% of those infected by genotypes 2 and 3. Beside the limited efficacy on HCV genotype 1, this combination therapy has significant side effects and is poorly tolerated in many patients. Major side effects include influenza-like symptoms, hematologic abnormalities, and neuropsychiatric symptoms. Hence there is a need for more effective, convenient and better-tolerated treatments.

Experience with HIV drugs, in particular with HIV protease inhibitors, has taught that sub-optimal pharmacokinetics and complex dosing regimes quickly result in inadvertent compliance failures. This in turn means that the 24 hour trough concentration (minimum plasma concentration) for the respective drugs in an HIV regime frequently falls below the IC₉₀ or ED₉₀ threshold for large parts of the day. It is considered that a 24 hour trough level of at least the IC₅₀, and more realistically, the IC₉₀ or ED₉₀, is essential to slow down the development of drug escape mutants. Achieving the necessary pharmacokinetics and drug metabolism to allow such trough levels provides a stringent challenge to drug design.

The NS5B RNA-dependent RNA polymerase (RdRp) is absolutely essential for replication of the single-stranded, positive sense, RNA genome. This enzyme has elicited significant interest among medicinal chemists. Both nucleoside and non-nucleoside inhibitors of NS5B are known.

Nucleoside inhibitors can act either as a chain terminator or as a competitive inhibitor, which interferes with nucleotide binding to the polymerase. To function as a chain terminator the nucleoside analog must be taken up by the cell and converted *in vivo* to a triphosphate to compete for the polymerase nucleotide binding site. This conversion to the triphosphate is commonly mediated by cellular kinases which imparts additional structural requirements on a potential nucleoside polymerase inhibitor. In addition this limits the direct evaluation of nucleosides as inhibitors of HCV replication to cell-based assays capable of *in situ* phosphorylation.

Several research groups have attempted to develop nucleosides as inhibitors of HCV polymerase, but while a handful of compounds have entered clinical development, none have proceeded all the way to registration. Amongst the problems that HCV targeted nucleosides to date have encountered are toxicity, mutagenicity, lack of selectivity, poor efficacy, poor bioavailability, sub-optimal dosage regimes and ensuing high pill burden and cost of goods.

Several patents and patent applications as well as scientific publications discloses nucleoside analogs having HCV inhibitory activity. WO 2007/020193 discloses phosphoramidate derivatives of certain nucleosides. WO 2008/043704 discloses 4-amino-1-((2R,3S,4S,5R)-5-azido-4-hydroxy-5-hydroxymethyl-3-methyltetrahydrofuran-2-yl)-1H-pyrimidin-2-one and ester derivatives as HCV polymerase inhibitors.

There is a need for HCV inhibitors that may overcome the disadvantages of current HCV therapy such as side effects, limited efficacy, the emerging of resistance, and compliance failures, as well as improve the sustained viral response.

The present invention concerns certain derivatives of 4-amino-1-((2R,3S,4S,5R)-5-azido-4-hydroxy-5-hydroxymethyl-3-methyl-tetrahydrofuran-2-yl)-1H-pyrimidin-2-one that are HCV inhibitors with useful properties as regards one or more of the parameters: antiviral efficacy, favorable profile of resistance development, lack of toxicity and genotoxicity, favorable pharmacokinetics and pharmacodynamics, such as an increased concentration of the mono or triphosphate analogs in the liver, increased absorption, in particular adsorption by liver cells, and ease of formulation and administration.

### Brief description of the invention

In accordance with the present invention, there is provided inhibitors of HCV polymerase, which can be represented by the formula I : 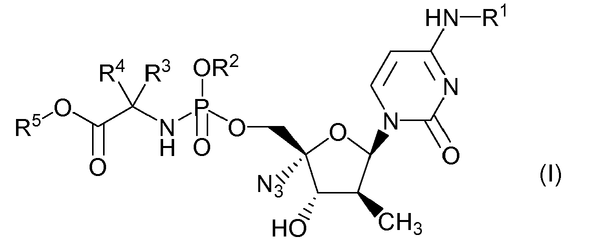 wherein:
R¹ is hydrogen, C₁-C₄alkyl, hydroxy, C₁-C₄alkoxy;
R² is phenyl, optionally substituted with 1, 2 or 3 substituents each independently selected from halo, C₁-C₆alkyl, C₂-C₆alkenyl, and C₁-C₆alkoxy, or R² is naphtalenyl;
R³ is hydrogen, C₁-C₆alkyl, benzyl;
R⁴ is hydrogen, C₁-C₆alkyl, benzyl; or
R³ and R⁴ together with the carbon atom to which they are attached form C₃-C₇cycloalkyl;
R⁵ is C₁-C₆alkyl, benzyl, or phenyl, optionally substituted with 1, 2 or 3 substituents each independently selected from hydroxy, C₁-C₆alkoxy, amino, mono- and diC₁-C₆alkylamino;
or a pharmaceutically acceptable salt or solvate thereof.

A further aspect of the invention provides a compound of formula I or a pharmaceutically acceptable salt, hydrate, or solvate thereof, for use in the treatment or prophylaxis of HCV infection. Or there is provided the use of a compound of formula I or a pharmaceutically acceptable salt, hydrate, or solvate thereof, for the manufacture of a medicament for the treatment or prophylaxis of HCV infection. Representative HCV genotypes in the context of treatment or prophylaxis in accordance with the invention include genotype 1a (prevalent in Europe) or 1b (prevalent in North America). In another aspect, the invention provides a method for the treatment or prophylaxis of HCV infection, in particular of the genotype 1a or 1b, said method comprising the administration of amount effective to treat HCV or to provide prophylaxis against HCV

### Detailed Description of the Invention

One subgroup of compounds comprises compounds of formula I, or any of the subgroups of compounds mentioned herein, wherein R¹ is hydrogen.

Another subgroup of compounds comprises compounds of formula I, or any of the subgroups of compounds mentioned herein, wherein R² is phenyl optionally substituted with 1, 2 or 3 substituents independently selected from halo, C₁-C₆alkyl, and C₂₋C₄alkenyl, or wherein R² is naphtalenyl. Of interest are compounds of formula I or any of the subgroups of compounds mentioned herein, wherein R² is phenyl, naphtalenyl or phenyl substituted with methyl, isopropyl and chloro, the latter more in particular being 2-methyl-3-chloro-5-isopropyl-phenyl.

In the compounds of formula I, or in any of the subgroups of compounds mentioned herein, the group -NH-C(R³)(R⁴)-CO- forms an amino acid residue, which includes natural and non-natural amino acid residues. Of particular interest are those amino acid residues wherein R³ is hydrogen. Where in the latter instance R⁴ is other than hydrogen, the configuration at the asymmetric carbon atom bearing R³ and R⁴ preferably is that of an L-amino acid. Examples of -NH-C(R³)(R⁴)-CO- are glycine (Gly), alanine (Ala), 1,1-dimethylglycine, valine (Val), isoleucine (Ile) and phenylalanine (Phe) residues, in particular the L-form such as L-Ala, L-Val, L-Ile, and L-Phe. An example of an amino acid residue wherein R³ and R⁴ together with the carbon atom to which they are attached form C₃-C₇cycloalkyl, is 1,1-cyclopropylamino acid.

One subgroup of compounds comprises compounds of formula I, or any of the subgroups of compounds mentioned herein, wherein R³ is hydrogen or C₁-C₆alkyl, and R⁴ is hydrogen or C₁-C₆alkyl; or wherein R³ is hydrogen or C₁-C₄alkyl, and R⁴ is hydrogen or C₁-C₄alkyl; or wherein R³ is hydrogen, and R⁴ is hydrogen or C₁-C₄alkyl. A particular subgroup amongst the foregoing is that wherein R³ is hydrogen and wherein the carbon bearing R³ and R⁴ is in L-configuration. In one embodiment, in the compounds of formula I, or in any of the subgroups of compounds mentioned herein, R³ is hydrogen and R⁴ is methyl or a branched C₁-C₆alkyl, such as isopropyl or isobutyl. Of interest are the compounds of formula I, or any of the subgroups of compounds mentioned herein, wherein R³ is hydrogen and R⁴ is methyl, or wherein R³ is methyl and R⁴ is methyl. Also of interest are the compounds of formula I, or any of the subgroups of compounds mentioned herein, wherein the group -NH-C(R³)(R⁴)-CO-forms Gly, L-Ala, or α,α-dimethylglycine, in particular L-Ala or α,α-dimethylglycine, more in particular L-Ala.

In one embodiment in the compounds of formula I, or in any of the subgroups of compounds mentioned herein, R⁵ is C₁-C₆alkyl or benzyl, in particular R⁵ is methyl, ethyl, n.propyl or benzyl.

A particular subgroup of compounds of formula I are those wherein:
R¹ is hydrogen;
R² is phenyl substituted with 1, 2 or 3 substituents independently selected from
C₁-C₆alkyl, halo, and C₂-C₄alkenyl;
-NH-C(R³)(R⁴)-CO- forms L-Ala or α,α-dimethylglycine;
R⁵ is C₁-C₄alkyl or benzyl.

A particular subgroup of compounds of formula I are those wherein:
R¹ is hydrogen;
R² is phenyl, phenyl substituted with two C₁-C₄alkyl and with halo, or naphtalenyl;
-NH-C(R³)(R⁴)-CO- forms L-Ala or α,α-dimethylglycine;
R⁵ is C₁-C₄alkyl or benzyl.

The compounds of formula I have several chiral centers and are represented herein as a specific stereoisomer. This also applies to some of the intermediates used in the preparation of the compounds of formula I, which intermediates may contain one or more chiral centers. However, the compounds of formula I, or any of the intermediates used in their preparation that have at least one chiral center, may contain small amounts of the other stereoisomers, i.e. stereoisomers with different chirality at one or more of the asymmetric centers. The total amount of the other stereoisomers in particular does not exceed 25%, or 20%, or 10%, or 5%, or 2%, or 1%, or 0.5%, or 0.1% by weight.

The absolute configuration at each of the chiral centers can be determined using art-known methods such as, for example, X-ray diffraction or NMR and/or by implication from starting materials of known stereochemistry. Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphorsulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound are synthesized by stereospecific methods of preparation. These methods will advantageously employ enantiomerically pure starting materials.

The diastereomeric racemates of the compounds of formula I can be obtained separately by conventional methods. Appropriate physical separation methods that may advantageously be employed are, for example, selective crystallization and chromatography, e.g. column chromatography.

The pharmaceutically acceptable addition salts comprise the therapeutically active non-toxic acid and base addition salt forms of the compounds of formula I. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxyl-butanedioic acid), tartaric, citric, methanesulfonic, ethanesulfonic, benzenesulfonic, *p*-toluenesulfonic, cyclamic, salicylic, *p*-aminosalicylic, pamoic and the like acids.

Conversely said salt forms can be converted by treatment with an appropriate base into the free base form.

The compounds of formula I that contain an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term "solvates" refers any solvates that the compounds of formula I as well as the salts thereof, may form. Such solvates are for example hydrates, alcoholates, e.g. ethanolates, propanolates, including n.propanolates and isopropanolates, and the like.

Some of the compounds of formula I may also exist in their tautomeric form. The uridine base is an example of such a form. Such forms although not explicitly indicated in the above formula are intended to be included within the scope of the present invention.

As used herein 'C₁₋C₄alkyl' as a group or part of a group defines saturated straight or branched chain hydrocarbon radicals having from 1 to 4 carbon atoms such as for example methyl, ethyl, 1-propyl, 2-propyl, 1-butyl, 2-butyl, 2-methyl-1-propyl, 2-methyl-2-propyl. 'C₁-C₆alkyl' encompasses C₁-C₄alkyl radicals and the higher homologues thereof having 5 or 6 carbon atoms such as, for example, 1-pentyl, 2-pentyl, 3-pentyl, 1-hexyl, 2-hexyl, 2-methyl-1-butyl, 2-methyl-1-pentyl, 2-ethyl-1-butyl, 3-methyl-2-pentyl, and the like. Of interest amongst C₁-C₆alkyl is C₁-C₄alkyl. 'C₂₋C₆alkenyl' as a group or part of a group defines saturated straight or branched chain hydrocarbon radicals having from 1 to 6 carbon atoms and one double bond such as for example ethenyl, 1-propenyl, 2-propenyl, 1-butenyl, 2-butenyl, 3-butenyl, 2-methyl-1-propenyl, 2-methyl-2-propenyl, 4-pentenyl, 3-pentenyl, 5-hexenyl, 4-hexenyl, 3-hexenyl, and the like. One subgroup of C₂₋C₆alkenyl includes the C₂₋C₆alkenyl radicals in which the carbon with which the group is linked to the remainder of the molecule is saturated. Another subgroup are the C₂₋C₄alkenyl, which are alkenyl radicals with two to four carbon atoms. Other subgroups of C₂₋C₆alkenyl include the C₂₋C₆alkenyl or the radicals C₂₋C₄alkenyls in which the carbon with which the group is linked to the remainder of the molecule is saturated. 'C₁-C₄alkoxy' refers to a radical -O-C₁-C₄alkyl wherein C₁-C₄alkyl is as defined above. C₁-C₄alkoxy radicals of interest include but are not limited to methoxy, ethoxy, n-propoxy and isopropoxy.

"C₃-C₆ cycloalkyl" includes cyclopropyl, cyclobutyl, cyclopentyl and cyclohexyl.
Of interest is cyclopropyl.

The term 'halo' is generic to fluoro, chloro, bromo and iodo.

As used herein, the term '(=O)' or 'oxo' forms a carbonyl moiety when attached to a carbon atom. It should be noted that an atom can only be substituted with an oxo group when the valency of that atom so permits.

It should be noted that the radical positions on any molecular moiety used in the definitions may be anywhere on such a moiety as long as it is chemically stable. When any variable occurs more than one time in any moiety, each definition is independent.

Whenever used herein, the term 'compounds of formula I', or 'the present compounds' or similar terms, it is meant to include the compounds of formula I, as well as their salts and solvates.

The compounds of formula I can be prepared by reacting a nucleoside derivative (2) with a chlorophosphoramidate (1). This reaction is conducted in a reaction-inert solvent such as an ether, e.g. diethylether, THF, or a halogenated hydrocarbon, e.g. dichloromethane, in the presence of a base.

Where R is hydrogen and the group -NH-R¹ is amino, the latter preferably is protected by a N-protecting group, in particular by reacting the free amino with *N,N*-dimethylformamide dimethyl acetal.

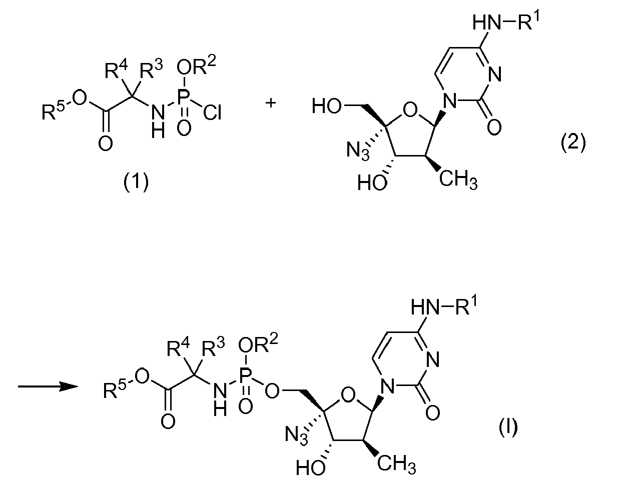

The nucleoside derivative (2) can be prepared as described in WO 2008/043704. The chlorophosphoramidate (1) can be prepared starting from POCl₃, which is reacted with a phenol or naphtalenol R²-OH and subsequently with an amino acid derivative H₂N-C(R³)(R⁴)-CO-OR⁵. R⁵ may be as defined above, but may also be a carboxyl acid protecting group that is removed and replaced by the desired R⁵ group. These reactions are conducted in a reaction inert solvent, such as the solvents mentioned above in relation to the preparation of the compounds of formula I.

In a further aspect, the present invention concerns a pharmaceutical composition comprising a therapeutically effective amount of a compound of formula I as specified herein, and a pharmaceutically acceptable carrier. A therapeutically effective amount in this context is an amount sufficient to act in a prophylactic way against, to stabilize or to reduce viral infection, and in particular HCV viral infection, in infected subjects or subjects being at risk of being infected. In still a further aspect, this invention relates to a process of preparing a pharmaceutical composition as specified herein, which comprises intimately mixing a pharmaceutically acceptable carrier with a therapeutically effective amount of a compound of formula I, as specified herein.

To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

The compounds of the present invention may also be administered via oral inhalation or insufflation by means of methods and formulations employed in the art for administration via this way. Thus, in general the compounds of the present invention may be administered to the lungs in the form of a solution, a suspension or a dry powder, a solution being preferred. Any system developed for the delivery of solutions, suspensions or dry powders via oral inhalation or insufflation are suitable for the administration of the present compounds.

Thus, the present invention also provides a pharmaceutical composition adapted for administration by inhalation or insufflation through the mouth comprising a compound of formula I and a pharmaceutically acceptable carrier. Preferably, the compounds of the present invention are administered via inhalation of a solution in nebulized or aerosolized doses.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compounds of formula I show antiviral properties. Viral infections and their associated diseases treatable using the compounds and methods of the present invention include those infections brought on by HCV and other pathogenic flaviviruses such as Yellow fever, Dengue fever (types 1-4), St. Louis encephalitis, Japanese encephalitis, Murray valley encephalitis, West Nile virus and Kunjin virus. The diseases associated with HCV include progressive liver fibrosis, inflammation and necrosis leading to cirrhosis, end-stage liver disease, and HCC; and for the other pathogenic flaviruses the diseases include yellow fever, dengue fever, hemorraghic fever and encephalitis. A number of the compounds of this invention moreover are believed to be active against mutated strains of HCV.

Additionally, many of the compounds of this invention show a favorable pharmacokinetic profile and have attractive properties in terms ofbioavailabilty, including an acceptable half-life, AUC (area under the curve) and peak values and lacking unfavorable phenomena such as insufficient quick onset and tissue retention.

The *in vitro* antiviral activity against HCV of the compounds of formula I can be tested in a cellular HCV replicon system based on Lohmann et al. (1999) Science 285:110-113, with the further modifications described by Krieger et al. (2001) Journal of Virology 75: 4614-4624 (incorporated herein by reference), which is further exemplified in the examples section. This model, while not a complete infection model for HCV, is widely accepted as the most robust and efficient model of autonomous HCV RNA replication currently available. Compounds exhibiting anti-HCV activity in this cellular model are considered as candidates for further development in the treatment of HCV infections in mammals. It will be appreciated that it is important to distinguish between compounds that specifically interfere with HCV functions from those that exert cytotoxic or cytostatic effects in the HCV replicon model, and as a consequence cause a decrease in HCV RNA or linked reporter enzyme concentration. Assays are known in the field for the evaluation of cellular cytotoxicity based for example on the activity of mitochondrial enzymes using fluorogenic redox dyes such as resazurin. Furthermore, cellular counter screens exist for the evaluation of non-selective inhibition of linked reporter gene activity, such as firefly luciferase. Appropriate cell types can be equipped by stable transfection with a luciferase reporter gene whose expression is dependent on a constitutively active gene promoter, and such cells can be used as a counter-screen to eliminate non-selective inhibitors.

Due to their antiviral properties, particularly their anti-HCV properties, the compounds of formula I and the pharmaceutically acceptable salts or solvates thereof, are useful in the treatment of individuals infected with a virus, particularly a virus that is HCV , and for the prophylaxis of viral infections, in particular HCV infections. In general, the compounds of the present invention may be useful in the treatment of warm-blooded animals infected with viruses, in particular flaviviruses such as HCV.

The compounds of the present invention may therefore be used as a medicine. The present invention also relates to the use of the compounds of the present invention in the manufacture of a medicament for the treatment or the prevention of a viral infection, particularly HCV infection.

The present invention furthermore relates to a method of treating a warm-blooded animal infected by a virus, or being at risk of infection by a virus, in particular by HCV, said method comprising the administration of an anti-virally effective amount of a compound of formula I, as specified herein. Said use as a medicine or method of treatment comprises the systemic administration to virally infected subjects or to subjects susceptible to viral infections of an amount effective to combat the conditions associated with the viral infection, in particular HCV infection.

In general it is contemplated that an antiviral effective daily amount would be from 0.01 mg/kg to 500 mg/kg body weight, or from 0.1 mg/kg to 50 mg/kg body weight, or from 0.5 mg/kg to 5 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The invention also relates to a combination of a compound of formula I, a pharmaceutically acceptable salt, or a pharmaceutically acceptable solvate thereof, and another antiviral compound, in particular another anti-HCV compound. The term "combination" may relate to a product containing (a) a compound of formula I, as specified above, and (b) optionally another anti-HCV compound, as a combined preparation for simultaneous, separate or sequential use in treatment of HCV infections.

Anti-HCV compounds that can be used in such combinations include nucleoside or nun-nucleoside HCV polymerase inhibitors, HCV protease inhibitors, HCV helicase inhibitors, or HCV fusion inhibitors. Other agents that can be used in such combinations include interferon-α (IFN-α), pegylated interferon-α, and ribavirin.

Any of the above-mentioned combinations may be formulated in the form of a pharmaceutical composition that includes the active ingredients described above and a carrier, as described above. Each of the active ingredients may be formulated separately and the formulations may be co-administered, or one formulation containing both and if desired further active ingredients may be provided. In the former instance, the combinations may also be formulated as a combined preparation for simultaneous, separate or sequential use in HCV therapy. The said composition may take any of the forms described above. In one embodiment, both ingredients are formulated in one dosage form such as a fixed dosage combination.

The individual components of the combinations of the present invention can be administered separately at different times during the course of therapy or concurrently in divided or single combination forms. Preferably, the separate dosage forms are administered simultaneously.

The compounds of formula I, or the combinations described herein, including those with other anti-HCV agents, may also be combined with an agent that has a positive effect on drug metabolism and/or pharmacokinetics that improve bioavailabilty, e.g. ritonavir or a pharmaceutically acceptable salt thereof. The ritonavir may be used as separate formulation, or may be co-formulated with one or more of the active agents of the combinations of the present invention. The weight ratio of the compound of formula I to ritonavir may be in the range of from about 10:1 to about 1:10, or from about 6:1 to about 1:6, or from about 1:1 to about 10:1, or from about 1:1 to about 6:1, or from about 1:1 to about 4:1, or from about 1:1 to about 3:1, or from about 1:1 to about 2:1.

### Examples

The following examples are meant to illustrate the invention and not to limit its scope thereto. The symbol Bn represents benzyl.

### Example 1

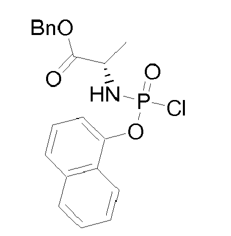

3g naphtol were loaded into a 3-neck flask and dissolved in Et₂O (60 mL) under N₂. To the solution was added POCl₃ (1 eq.) and the resulting solution cooled to -78 °C. To the cold solution was added drop wise (over approx. 15min.) Et₃N (1 eq.). The reaction was allowed to reach rt overnight and then the white solid was filtered off washing with Et₂O and avoiding contact with moisture. The combined ethereal phases were concentrated in vacuum and the residue re-dissolved in CH₂Cl₂ (120mL) under N₂. To the solution was added L-alanine benzyl ester hydrochloride (1 eq.) and the mixture cooled to -78 °C. To this was then added drop wise (over approx. 45min) Et₃N (1 eq.) and the reaction allowed to reach rt overnight. The solvent was removed in vacuum, avoiding contact with moisture and the residue passed through dry silica-gel eluting with EtOAc/heptane:70/30 and protecting the collected fractions from moisture. The fractions containing the product were concentrated in vacuum avoiding contact with moisture and the residue dissolved in dry THF as to obtain a standard solution of approx. concentration used as such in the next reaction.

### Example 2

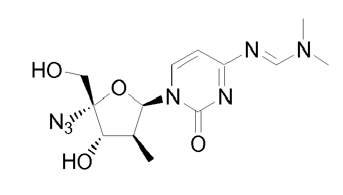

The corresponding Cyt compound (408mg, 1.44mmol, approx. 81% pure) was dissolved under N₂ in HPLC grade MeOH (10mL). *N, N*-dimethylformamide dimethyl acetal (10 eq.) was added to the suspension. A clear solution was almost immediately obtained and stirred at room temperature for 4h or until completion of the reaction as monitored by LC-MS. The solvent was removed and the residue used as such in the next step.

### Example 3

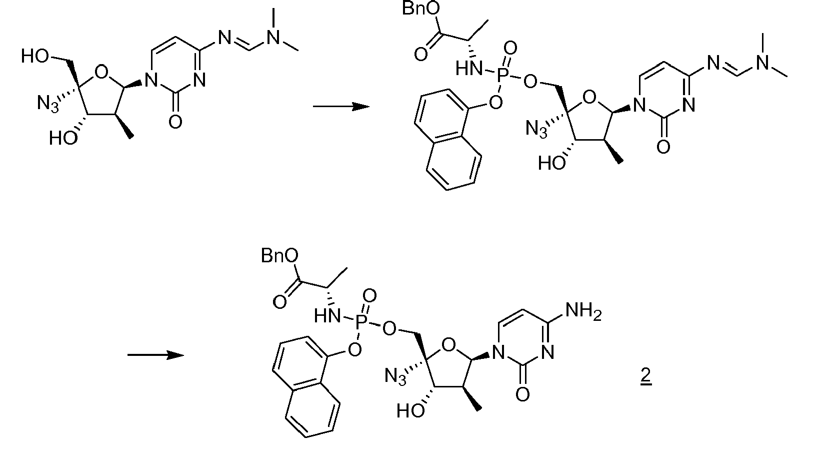

### Step 1

The compound prepared in example 2 was dissolved in CH₂Cl₂ (12mL) and loaded into a 3-neck flask under N₂. The solution was cooled to -78 °C and 2 to 3 eq. of the THF solution of the phosphorous reagent prepared above added slowly. To this new solution was then added drop wise *N*-methyl imidazole (8 eq.). The reaction was allowed to reach rt and stirred at this temperature. LC-MS after 150min showed no starting material. The LC trace looks complex, but it clearly shows the product, no s.m. and no product of reaction at 3'. The solvents were removed in vacuum and the residue re-dissolved in CH₂Cl₂ (50mL). The latter solution was quickly washed with 0.5M HCl and brine, dried over Na₂SO₄, filtered, concentrated and submitted without further delay to purification by flash chromatography on silica-gel. Gradient CH₂Cl₂/MeOH: 100/0 to 95/5 to 90/10, Rf in 95/5 = 0.17. The intermediate (mass yield = 20%) obtained in this way is approx. 51 % pure and was used in the next step as such.

### Step 2

The compound obtained in step 1 was dissolved in CH₃CN/0.1MHCl: 1/1 (approx. 10mL). To the solution was added drop wise 1M HCl until pH = 2 and the resulting solution stirred for 24h. After this time the pH was adjusted to neutral by drop wise addition of sat. NaHCO₃. The mixture was evaporated to dryness and the residue taken into CH₃CN (3mL) and filtered through a syringe filter to remove insoluble materials. Compound 2 was obtained in 19% yield by preparative HPLC on C-8. Yield from the parent Cyt. compound was approx. 3.7%. LR-MS: calculated: 649.2, found: 649.2. Purity: 95.3%. ¹H NMR ([CDCl₃): δ = 8.09 (d, 1 H), 7.87 (d, 1 H), 7.68 (d, 1 H), 7.59-7.52 (m, 4 H), 7.41-7.28 (5, 1 H), 7.11 (d, 1 H), 6.53 (bs, 1 H), 5.27 (m, 2 H), 5.11 (bs, 1 H), 4.50 (q, 2 H), 4.38 (bs, 1 H), 4.18 (m, 1 H), 3.90 (bs, 1 H), 2.72 (m, 1 H), 1.39 (t, 3 H), 0.91 (d, 3 H) ppm

### Example 4

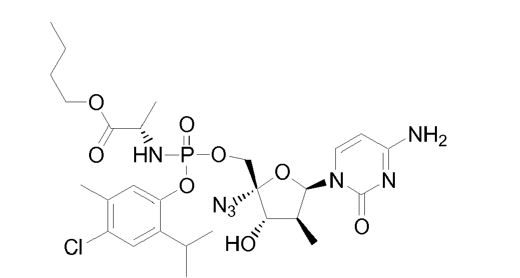

Step a) 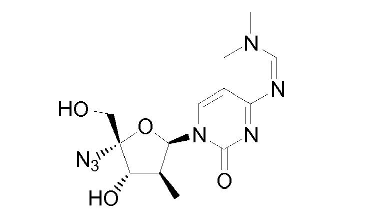
4'-azido-2'deoxy-2'-β-methylcytidine (1.05mmol) was dissolved in HPLC grade MeOH under N₂. To the solution was added *N,N*-dimethylformamide dimethylacetal (10eq.) and the reaction allowed to proceed at rt for 2h. After this time volatiles were removed and the residue purified by flash chromatography on silica-gel using gradient CH₂Cl₂/MeOH:100/0 to 95/5 to 90/10 to afford the above compound (0.95mmol) in 91 % yield.
Step b) 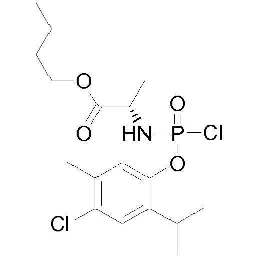 Commercially available chlorothymol, i.e. 4-chloro-2-isopropyl-5-methylphenol (8.73mmol) was dissolved in dry Et₂O (18mL) under N₂. To the solution was added POCl₃ (1eq.) and the resulting solution cooled to -78°C. To the cold mixture was then added drop wise Et₃N (1eq.) and the reaction allowed to reach rt overnight. The formed salt was removed by filtration avoiding contact with moisture and washed with dry Et₂O. The combined ether phases were concentrated in vacuum and the residue re-dissolved in CH₂Cl₂ (15mL) under N₂. To the solution was added (L)-alanine *n*-butyl ester hydrochloride (1eq. prepared from (L)-alanine, SOCl₂ and *n*-butanol) and the mixture cooled to -78°C. To the cold mixture was then added Et₃N (1eq.) drop wise over 30min and the reaction allowed to reach rt overnight. The solvent was stripped off and replaced by EtOAc/heptane:40/60 (approximately 20mL) and transferred via syringe into a column packed with dry silica-gel (freeze dried for 2 days prior to use) and the same solvent mixture. Flash chromatography was performed using the same solvent mixture and N₂ as pressure gas, the fractions were collected into vials that were caped under N₂ and analyzed by TLC. The fractions containing the product were combined and concentrated by evaporation always keeping inert atmosphere during transfers to afford 2.80mmol of the pure desired reagent (32% yield). This was dissolved in dry CH₂Cl₂ to give a 0.15M solution in this solvent that was kept in a Schlenk-flask at +4°C.
Step c)

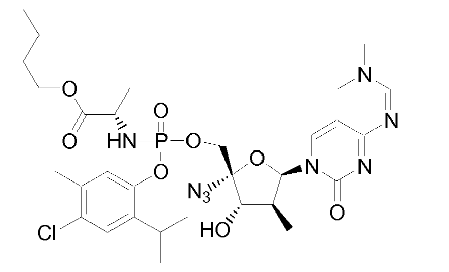

The N-protected 4'-azido, 2'-deoxy-2'-β-methylcytidine from step a) (0.95mmol) was suspended in CH₂Cl₂ (10mL) under N₂. To it was added *N*-methyl imidazole (4eq.) and the resulting solution cooled to -78°C. To this mixture was then added drop wise a solution (0.15M) of the phosphorous reagent prepared above (1.25eq.) and the reaction stirred cold for 90min and then at rt for 26h (complete conversion according to LC-MS). The reaction was diluted with CH₂Cl₂ (20mL) and quickly washed with 0.5M HCl, sat.NaHCO₃ and brine (10mL each). The organic layer was dried over Na₂SO₄ and concentrated in vacuum. The residue was purified by flash chromatography on silica-gel eluting with CH₂Cl₂/MeOH: 96/4 to give the above compound in 11 % yield.
Step d)

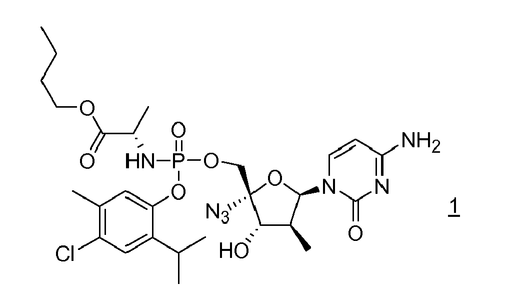

Deprotection of the compound of step c) (0.10mmol) was carried out by dissolving it in CH₃CN/0.01M HCl:1/1 (4mL) and adding drop wise 0.1M HCl until pH = 2. The solution was then stirred at rt for 20h and made basic by addition of an excess Et₃N. Evaporation of the solvents afforded a residue that was purified by preparative HPLC on a C-8 column to give compound 1 in 28% yield. LC-UV/MS 655.2 [M+H]⁺

### Biological Examples

### Replicon assay

The compounds of formula I are examined for activity in the inhibition of HCV RNA replication in a cellular assay. The assay demonstrates that the compounds of formula I exhibit activity against HCV replicons functional in a cell culture. The cellular assay can be based on a bicistronic expression construct, as described by Lohmann et al. (1999) Science vol. 285 pp. 110-113 with modifications described by Krieger et al. (2001) Journal of Virology 75: 4614-4624, in a multi-target screening strategy. The Bartenschlager replicon assays are available commercially from ReBLikon GmbH in Mainz,Germany.

In essence, the method is as follows.
The assay utilized the stably transfected cell line Huh-7 luc/neo (hereafter referred to as Huh-Luc). This cell line harbors an RNA encoding a bicistronic expression construct comprising the wild type NS3-NS5B regions of HCV type 1b translated from an Internal Ribosome Entry Site (IRES) from encephalomyocarditis virus (EMCV), preceded by a reporter portion (FfL-luciferase), and a selectable marker portion (neo^{R}, neomycine phosphotransferase). The construct is bordered by 5' and 3' NTRs (non-translated regions) from HCV type 1b. Continued culture of the replicon cells in the presence of G418 (neo^{R}) is dependent on the replication of the HCV RNA. The stably transfected replicon cells that express HCV RNA, which replicates autonomously and to high levels, encoding *inter alia* luciferase, are used for screening the antiviral compounds.

The replicon cells were plated in well plates in the presence of the test and control compounds, which were added in various concentrations. Following an incubation of three days, HCV replication was measured by assaying luciferase activity (using standard luciferase assay substrates and reagents and a Perkin Elmer ViewLux^{Tm} ultraHTS microplate imager). Replicon cells in the control cultures have high luciferase expression in the absence of any inhibitor. The inhibitory activity of the compound on luciferase activity was monitored on the Huh-Luc cells, enabling a dose-response curve for each test compound. EC50 values were then calculated, which value represents the amount of the compound required to decrease by 50% the level of detected luciferase activity, or more specifically, the ability of the genetically linked HCV replicon RNA to replicate.

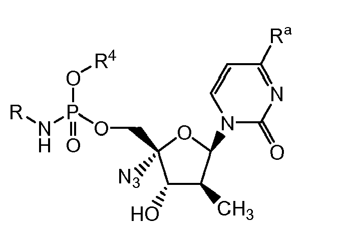

| Cpd. No | R | R⁴ | R^{a} | EC₅₀ |
|---|---|---|---|---|
| 1 | 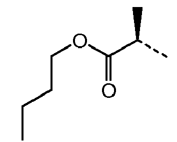 | 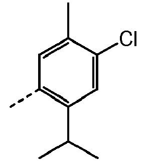 | --NH₂ | = 0.74 |
| 2 | 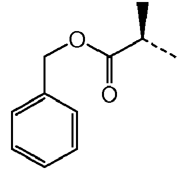 | 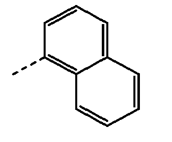 | --NH₂ | = 0.89 |
| 3 | 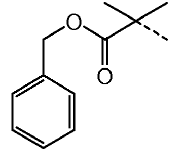 | 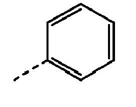 | --NH₂ | = 2.19 |
| 4 | 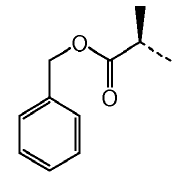 | 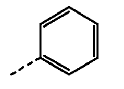 | --NH₂ | = 4.82 |
| 5 | 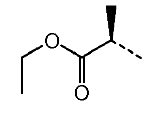 | 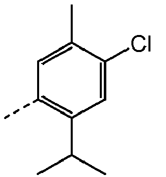 | --NH₂ | = 4.87 |
| 6 | 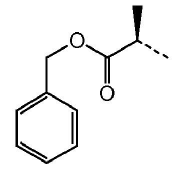 | 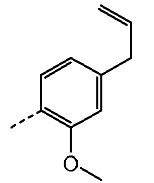 | --NH₂ | = 4.98 |
| 7 | 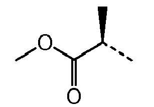 | 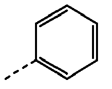 | --NH₂ | = 11.46 |
| 8 | 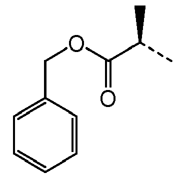 | 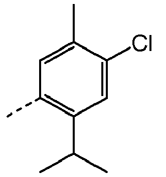 | --NH₂ | = 11.64 |
| 9 | 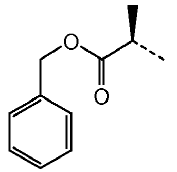 | 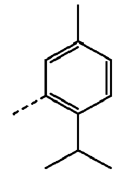 | --NH₂ | = 19.65 |
| 10 | 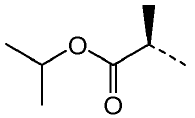 | 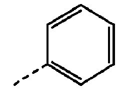 | --NH₂ | = 27.10 |
| 11 | 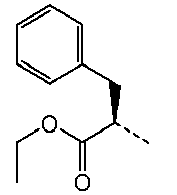 | 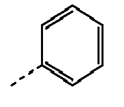 | --NH₂ | > 32.00 |

### Inhibition assay

Enzyme assays with various HCV NS5 constructs are described in EP 842276, EP 858833 and EP1037974. Polymerase assays typically employ the polymerase, a pool of nucleotide triphosphates and a primer/template. Nucleoside test compounds must generally be synthetically phosphorylated to the triphosphate, which is an arduous procedure. EP1350276 describes a reporter gene assay intended to avoid that disadvantage of isolated enzyme assays.

## Claims

1. A compound the formula I : 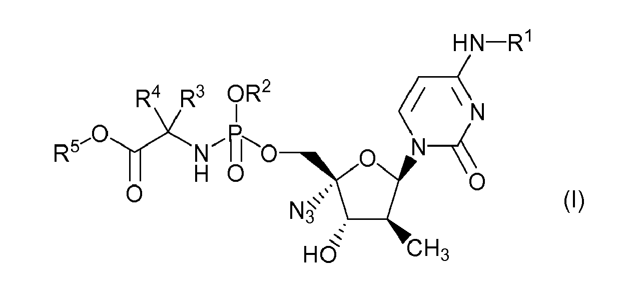 wherein:
R¹ is hydrogen, C₁-C₄alkyl, hydroxy, C₁-C₄alkoxy;
R² is phenyl, optionally substituted with 1, 2 or 3 substituents each independently selected from halo, C₁-C₆alkyl, and C₁-C₆alkoxy, or R² is naphtalenyl;
R³ is hydrogen, C₁-C₆alkyl, benzyl;
R⁴ is hydrogen, C₁-C₆alkyl, benzyl; or
R³ and R⁴ together with the carbon atom to which they are attached form C₃-C₇cycloalkyl;
R⁵ is C₁-C₆alkyl, benzyl, or phenyl, optionally substituted with 1, 2 or 3 substituents each independently selected from hydroxy, C₁-C₆alkoxy, amino, mono- and diC₁-C₆alkylamino;
or a pharmaceutically acceptable salt or solvate thereof.

2. A compound according to claim 1, wherein R¹ is hydrogen.

3. A compound according to any preceding claim, wherein R² is phenyl optionally substituted with 1, 2 or 3 substituents independently selected from halo, C₁-C₆alkyl, and C₂₋C₄alkenyl, or wherein R² is naphtalenyl.

4. A compound according to any preceding claim, wherein R³ is hydrogen and R⁴ is meth or wherein R³ and R⁴ are both methyl.

5. A compound according to any preceding claim, wherein R⁵ is n. propyl or benzyl.

6. A compound according to claim 1, wherein R¹ is hydrogen;
R² is phenyl substituted with 1, 2 or 3 substituents independently selected from
C₁-C₆alkyl, halo, and C₂-C₄alkenyl;
-NH-C(R³)(R⁴)-CO- forms L-Ala or α,α-dimethylglycine;
R⁵ is C₁-C₄alkyl or benzyl.

7. A compound according to claim 1, wherein R¹ is hydrogen;
R² is phenyl, phenyl substituted with two C₁-C₄alkyl and with halo, or naphtalenyl;
-NH-C(R³)(R⁴)-CO- forms L-Ala or α,α-dimethylglycine;
R⁵ is C₁-C₄alkyl or benzyl.

8. A pharmaceutical composition comprising a compound of formula I as defined in any of claims 1 - 7 and a pharmaceutically acceptable carrier.

9. A pharmaceutical composition according to claim 8, further comprising at least one additional HCV antiviral.

10. Use of a compound according to any one of claims 1 to 7 in the manufacture of a medicament for the treatment or prophylaxis of HCV infection.
